# FASCICULE DE BREVET EUROPEEN

(11) **EP 1 061 909 B1**
(45) Date de publication et mention de la délivrance du brevet: **05.06.2002**
(21) Numéro de dépôt: 99907688.8
(22) Date de dépôt: 11.03.1999
(51) Int. Cl.: A61K 31/195, A61K 31/19, A61K 31/13, A61P 1/00, A61P 13/00

(54) **SELS DE CETOACIDES ET DE DERIVES AMINES, ET LEUR UTILISATION POUR LA PREPARATION DE MEDICAMENTS**
SALZE DER KETOSÄUREN UND AMINE SOWIE IHRE VERWENDUNG ZUR HERSTELLUNG VON MEDIKAMENTEN
KETO ACID SALTS AND AMINE DERIVATIVES, AND THEIR USE FOR PREPARING MEDICINES

(30) Priorité: 13.03.1998 FR 9803155
(43) Date de publication de la demande: 27.12.2000
(73) Titulaire: CHIESI S.A., 92400 Courbevoie (FR)
(72) Inventeur: BOUYSSOU, Thierry, F-78650 Beynex (FR); BIOSA, Serge, F-78200 Mantes la Jolie (FR); SETTEMBRE, Pierre-André, F-78800 Houilles (FR); JEANPETIT, Christian, F-78380 Bougival (FR)
(74) Mandataire: Grosset-Fournier, Chantal Catherine
(86) Numéro de dépôt international: FR9900545
(87) Numéro de publication internationale: WO9947134

(56) Documents cités:
- FR-M- 3 533
- US-A- 4 320 146
- US-A- 4 734 276
- US-A- 4 957 938

## Description

La présente invention a pour objet des sels de cétoacides et de dérivés aminés, ainsi que leur utilisation pour la préparation de compositions pharmaceutiques destinées au traitement de pathologies dans lesquelles sont impliqués les neurones silencieux.

Le message nociceptif résulte de l'activation intense des terminaisons libres (nocicepteurs) des fibres C et Aδ qui dans les conditions physiologiques participent à la régulation du fonctionnement des organes. Ces fibres sont contenues dans les tissus cutanés, musculaires, articulaires ainsi que dans la paroi des viscères.

Dans le tube digestif ainsi que dans la vessie et les voies biliaires, cette structure nerveuse cohabite avec une population de neurones "silencieux" (Mayer, E.A. ; Gebhart, G.F. ; Gastoenterology, 1994, 107, 271-293) dont l'expression n'apparaît qu'en présence d'une lésion inflammatoire ou nerveuse.

Ce mécanisme physiopathologique a inspiré la mise en place du modèle de distension du côlon préalablement irrité à l'acide acétique 1 % chez le rat. Ce modèle est donc représentatif des douleurs d'origine digestive et est utilisé notamment pour la mise en évidence de composés actifs dans le traitement du syndrome de l'intestin irritable (SII). Le SII est caractérisé par la présence de douleurs abdominales. Les patients atteints de cette pathologie présentent un seuil de sensibilité digestive abaissé.

La morphine est active sur le modèle de distension colique alors que les analgésiques classiques (AINS, aspirine, paracétamol) sont inactifs sur le test. Ils sont en revanche actifs sur le "writhing test" (modèle de douleur viscérale dans laquelle ne sont pas impliqués les neurones silencieux) qui est réalisé par injection intrapéritonéale de phénylbenzoquinone ou d'acide acétique à 3 % chez le rat ou la souris. La morphine est donc capable de modifier la réactivité des nocicepteurs silencieux, ce que ne peuvent pas faire les AINS.

De façon analogue l'ornithine, décrite dans le brevet JP 043 05524 en tant qu'analgésique, est active sur le "writhing test" à 500 et 1 000 mg/kg par voie intraveineuse sur la souris. Elle est également active sur un test de douleur somatique dans laquelle ne sont pas impliqués les neurones silencieux (Kawabata, Atsugumi et coll., Eur. J. Pharmacol., 1996, 296 (1), 23-31), à des doses situées entre 300 et 1 000 mg/kg par voie sous-cutanée sur le rat.

Par contre, elle est inactive sur le modèle de distension colique chez le rat à des doses de 1 à 20 mg/kg par voie orale (tableau I). L'ornithine n'est donc pas capable de modifier la réactivité des nocicepteurs silencieux à ces doses.

Par ailleurs, J. Goldhill et coll. (Gastroenterology, Avril 1996 , 110 (4), abstract A916) ont montré que la glutamine était active sur le test de distension colique à la dose de 6 mg/kg par voie rectale chez le rat (administration locale sur la muqueuse colique irritée).

Enfin l'α-cétoglutarate de di-ornithine est utilisé en thérapeutique (brevet français n°3 533 M) pour améliorer le métabolisme protéique de sujets dénutris. Il est également connu comme stimulant hormonal (hormone de croissance et insuline) et stimulant de la croissance cellulaire.

La présente invention découle de la mise en évidence par les Inventeurs, du fait que des sels de formule générale (I):

(X)ₙ₁,Y,(Z)ₙ₂ (I)

dans laquelle :
X est un acide aminé ;
Y est un cétoacide ;
Z est un acide aminé ou une polyamine,
n₁ et n₂ représentent 0 ou 1, sous réserve que lorsque n₁ = 0 alors n₂ = 1, et que lorsque n₂ = 0 alors n₁ = 1,
ont la propriété d'augmenter le seuil de perception de la douleur digestive et par conséquent sont capables de modifier la réactivité des nocicepteurs silencieux à partir de 1 mg/kg par voie orale.

Cette mise en évidence est d'autant plus inattendue que les cétoacides testés séparément sont inactifs. Les amino-acides testés séparément sont également inactifs ou actifs à des doses supérieures (voir tableau I ci-après).

L'activité de ces sels n'est pas due au cétoacide seul ou à l'aminoacide seul, mais à une synergie entre ces deux types de composés.

Ainsi, à titre d'illustration, le tableau I, fait nettement apparaître que :
- les comparateurs 1 et 2 sont inactifs alors que les composés des exemples 1 et 2 (sels entre les comparateurs 1 et 2) sont actifs dès 1 mg/kg ;
- le comparateur 3 est inactif alors que le composé de l'exemple 6 (sel entre les comparateurs 3 et 2) est actif également dès 1 mg/kg ;
- le comparateur 4 est actif à 10 mg/kg alors que le composé de l'exemple 5 (sel entre les comparateurs 1, 2 et 4) est actif dès 1 mg/kg ;
- le comparateur 5 n'est actif qu'à 20 mg/kg alors que les composés des exemples 3 (sels des comparateurs 1 et 5) et 4 (sels des comparateurs 1, 2 et 5) sont actifs dès 10 mg/kg.

La présente invention a pour objet l'utilisation de composés de formule générale (I) suivante :

(X)ₙ₁,Y,(Z)ₙ₂ (I)

dans laquelle :
- n₁ et n₂ représentent 0 ou 1, sous réserve que lorsque n₁ = 0 alors n₂ = 1, et que lorsque n₂ = 0 alors n₁ = 1,
- X représente un aminoacide naturel, sous réserve que lorsque n₂ = 0 alors X représente un aminoacide basique tel que :
   - l'ornithine,
   - l'arginine,
   - la lysine,
   - ou, l'histidine,
- Y représente un cétoacide de formule (II) suivante :

   R-CO-COOH (II)

   dans laquelle R représente un groupe alkyle ou acide alcanoïque, substitué ou non, de 1 à 10 atomes de carbone, notamment un cétoacide de formule (II) dans laquelle lorsque R représente :
   - -CH₃, ledit cétoacide est l'acide pyruvique,
   - -CH₂-CH₃, ledit cétoacide est l'acide α-cétobutyrique,
   - -CH(CH₃)₂, ledit cétoacide est l'acide α-cétoisovalérique,
   - -CH(CH₃)-CH₂-CH₃, ledit cétoacide est l'acide α-céto β-méthylvalérique,
   - -CH₂-CH(CH₃)₂, ledit cétoacide est l'acide α-cétoisocaproïque,
   - -(CH₂)₂-COOH, ledit cétoacide est l'acide α-cétoglutarique,
   - -(CH₂)₃-COOH, ledit cétoacide est l'acide α-cétoadipique,
- Z représente :
   - un aminoacide naturel, notamment un aminoacide choisi parmi l'ornithine, l'arginine, la lysine, l'histidine, ou la glutamine,
   - ou, une polyamine comprenant au moins deux fonctions amines primaires, secondaires ou tertiaires, espacée par une chaîne hydrocarbonée linéaire ou ramifiée de 3 à 10 atomes de carbone, notamment une polyamine de formule (III) suivante :

      R₁-HN-(CH₂)n-NH-R₂ (III)

      dans laquelle :
      . n représente un nombre entier compris entre 4 et 5, et
         * lorsque n = 4,
            .. R₁ et R₂ représentent H,
            .. ou, R₁ représente H et R₂ représente (CH₂)₃NH₂,
            .. ou, R₁ et R₂ représentent (CH₂)₃NH₂,
         * lorsque n = 5,
            .. R₁ et R₂ représentent H,
            .. ou, R₁ représente H et R₂ représente un groupe de formule 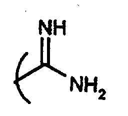
notamment une polyamine choisie parmi la cadavérine, la putrescine, la spermidine, la spermine ou l'agmatine,
pour la préparation d'un médicament destiné au traitement de pathologies humaines ou animales dans lesquelles sont impliqués les neurones silencieux, telles que les pathologies du tube digestif, de la vessie et des voies biliaires.

Il est bien entendu que les composés de formule (I) susmentionnée résultent de la formation de liaisons ioniques principalement, et en aucun cas de liaisons covalentes, entre les différents constituants X, Y ou Z. Par conséquent, l'ordre d'apparition de ces différents constituants dans la formule (I) n'a pas de signification particulière, et cette formule (I) doit être comprise comme comprenant aussi bien les composés de formule (X)ₙ₁,Y,(Z)ₙ₂, que ceux de formule (Z)ₙ₂,Y,(X)ₙ₁; (X)ₙ₁,(Z)ₙ₂,Y; (Z)ₙ₂,(X)ₙ₁,Y; Y,(X)ₙ₁,(Z)ₙ₂; Y,(Z)ₙ₂,(X)ₙ₁.

Avantageusement, l'invention a pour objet l'utilisation susmentionnée de composés de formule (I) telle que définie ci-dessus, dans laquelle Y représente un cétoacide choisi parmi l'acide α-cétoglutarique, ou l'acide α-cétobutyrique.

L'invention a plus particulièrement pour objet l'utilisation susmentionnée, de composés de formule (I) dans laquelle :
- n₁ = 1, et n₂ = 0 ou 1,
- X représente un acide aminé choisi parmi l'ornithine, l'arginine ou la glutamine,
- Y représente un cétoacide choisi parmi l'acide α-cétoglutarique, ou l'acide α-cétobutyrique,
- et, lorsque n₂ = 1, Z représente :
   . un acide aminé naturel, notamment l'ornithine, l'arginine ou la glutamine,
   . ou une polyamine de formule (III) susmentionnée, telle que la cadavérine, la putrescine, la spermidine, la spermine ou l'agmatine.

Des composés de formule (I) dans laquelle n₁ = 1, et n₂ = 0, particulièrement préférés pour leur utilisation dans le cadre de la présente invention, sont ceux pour lesquels :
- X représente l'ornithine et Y représente l'acide α-cétoglutarique, à savoir l'α-cétoglutarate de mono-ornithine,
- X représente l'ornithine et Y représente l'acide α-cétobutyrique, à savoir l'α-cétobutyrate de mono-ornithine,
ou ceux pour lesquels :
- X représente l'arginine et Y représente l'acide α-cétobutyrique, à savoir l'α-cétobutyrate d'arginine,
- X représente la lysine et Y représente l'acide α-cétobutyrique, à savoir l'α-cétobutyrate de lysine,
- X représente l'histidine et Y représente l'acide α-cétobutyrique, à savoir l'α-cétobutyrate d'histidine,
- X représente l'arginine et Y représente l'acide α-céto-isocaproïque, à savoir l'α-céto-isocaproate d'arginine,
- X représente l'ornithine et Y représente l'acide α-céto-isocaproïque, à savoir l'α-céto-isocaproate d'ornithine,
- X représente l'ornithine et Y représente l'acide α-céto-β méthylvalérique, à savoir l'α-céto-β méthylvalérate d'ornithine,
- X représente l'arginine et Y représente l'acide α-céto-β méthylvalérique, à savoir l'α-céto-β méthylvalérate d'arginine,
- X représente l'arginine et Y représente l'acide α-céto-isovalérique, à savoir l'α-céto-isovalérate d'arginine,
- X représente l'ornithine et Y représente l'acide α-céto-isovalérique, à savoir l'α-céto-isovalérate d'ornithine.

Des composés de formule (I) dans laquelle n₁ = 1, et n₂ = 1, particulièrement préférés pour leur utilisation dans le cadre de la présente invention, sont ceux pour lesquels :
- X représente l'ornithine, Y représente l'acide α-cétoglutarique, et Z représente l'ornithine, à savoir l'α-cétoglutarate de di-ornithine,
- X représente l'arginine, Y représente l'acide α-cétoglutarique, et Z représente l'arginine, à savoir l'α-cétoglutarate de di-arginine,
- X représente l'ornithine, Y représente l'acide α-cétoglutarique, et Z représente l'arginine, à savoir l'α-cétoglutarate d'ornithine et d'arginine,
- X représente l'ornithine, Y représente l'acide α-cétoglutarique, et Z représente la glutamine, à savoir l'α-cétoglutarate d'ornithine et de glutamine,
- X représente l'ornithine, Y représente l'acide α-cétoglutarique, et Z représente la spermidine, à savoir l'α-cétoglutarate d'ornithine et de spermidine.

De préférence, le composé utilisé dans le cadre de la présente invention est l'α-cétoglutarate de di-ornithine.

L'invention a plus particulièrement pour objet l'utilisation susmentionnée, de composés de formule (I) dans laquelle :
- n₁ = 0 ou 1, et n₂ = 1,
- lorsque n₁ = 1, X représente un aminoacide naturel, tel que l'omithine, l'arginine ou la glutamine,
- Y représente un cétoacide choisi parmi l'acide α-cétoglutarique, ou l'acide α-cétobutyrique,
- Z représente une polyamine de formule (III) susmentionnée, telle que la cadavérine, la putrescine, la spermidine, la spermine ou l'agmatine.

Un composé de formule (I) dans laquelle n₁ = 0, et n₂ = 1, particulièrement préféré pour son utilisation dans le cadre de la présente invention, est celui pour lequel Y représente l'acide α-cétoglutarique et Z représente la spermidine, à savoir l'α-cétoglutarate de spermidine.

L'invention a également pour objet les composés de formule générale (IV) suivante:

(X)ₙₐ,(Y)_{nb},Z (IV)

dans laquelle :
- nₐ et n_{b} représentent 0 ou 1, sous réserve que lorsque nₐ = 0 alors n_{b} = 1, et que lorsque n_{b} = 0 alors nₐ = 1,
- X représente un aminoacide naturel, notamment un aminoacide basique tel que l'ornithine, l'arginine, la lysine, ou l'histidine,
- Y représente un cétoacide de formule (II) susmentionnée,
- Z représente une polyamine comprenant au moins deux fonctions amines primaires, secondaires ou tertiaires, espacée par une chaîne hydrocarbonée linéaire ou ramifiée d'environ 3 à 10 atomes de carbone, notamment une polyamine de formule (III) susmentionnée, telle que la cadavérine, la putrescine, la spermidine, la spermine ou l'agmatine.

L'invention a plus particulièrement pour objet le composé de formule générale (IV) dans laquelle nₐ = 0 et n_{b} = 1, Y représente l'acide α-cétoglutarique, et Z représente la spermidine, et correspondant au composé de formule (I) dans laquelle n₁ = 0, et n₂ = 1, Y représente l'acide α-cétoglutarique et Z représente la spermidine, à savoir l'α-cétoglutarate de spermidine.

Avantageusement, les composés tels que décrits ci-dessus se présentent sous forme de sels entre deux constituants X et Y, ou Y et Z, ou entre les trois constituants X, Y et Z.

La proportion en poids des différents constituants est de préférence comprise entre 0,8 et 1,2, de sorte que la somme des proportions de chacun des constituants est égale à 2 dans un sel entre deux constituants X et Y, ou Y et Z, ou est égale à 3 dans un sel entre trois constituants X, Y et Z.

Avantageusement, la proportion susmentionnée des différents constituants est comprise entre 0,9 et 1,1.

Des composés particulièrement préférés sont ceux au sein desquels les différents constituants X et Y, ou Y et Z, ou X, Y et Z, sont dans un rapport équimolaire, c'est-à-dire que chacun des constituants est dans une proportion en poids de 1, de sorte que la somme des proportions de chacun des constituants est égale à 2 dans un sel entre deux constituants X et Y, ou Y et Z, ou est égale à 3 dans un sel entre les trois constituants X, Y et Z.

L'invention a plus particulièrement pour objet les composés choisis dans le groupe constitué par l'α-cétobutyrate d'arginine, l'α-cétobutyrate de lysine, l'α-cétobutyrate d'histidine, l'α-céto-isocaproate d'arginine, l'α-céto-isocaproate d'ornithine, l'α-céto-β méthylvalérate d'ornithine, l'α-céto-β méthylvalérate d'arginine, l'α-céto- isovalérate d'arginine, et l'α-céto- isovalérate d'ornithine.

L'invention a également pour objet toute composition pharmaceutique comprenant, à titre de principe actif, un composé de formule (I) décrite ci-dessus, en association avec un véhicule pharmaceutiquement acceptable.

L'invention concerne également toute composition pharmaceutique comprenant, à titre de principe actif, une polyamine de formule (III) décrite ci-dessus, et plus particulièrement la cadavérine, la putrescine, la spermidine, la spermine ou l'agmatine, ou un composé de formule (IV) susmentionnée, en association avec un véhicule pharmaceutiquement acceptable.

Les compositions pharmaceutiques selon l'invention se présentent sous une forme administrable par voie orale, parentérale, ou rectale.

De préférence, les compositions pharmaceutiques selon l'invention sont caractérisées en ce que la posologie en principe actif est d'environ 0,1 à 50 mg/kg/jour, de préférence 1 à 20 mg/kg/jour par voies orale et rectale, et d'environ 1 µg/kg/jour à 10 mg/kg/jour par voie parentérale.

Des compositions pharmaceutiques préférées selon l'invention se présentent sous une forme administrable par voie orale, en dose unitaire de 1 mg à 5 g de principe actif par prise et de préférence de 10 mg à 1 g à raison de 1 à 4 prises par jour. Des compositions également préférées selon l'invention se présentent sous une forme administrable par voie parentérale en dose unitaire de 50 µg à 500 mg de principe actif à raison de 1 à 2 injections par jour.

L'invention a plus particulièrement pour objet l'utilisation d'un ou plusieurs composés de formule (I) décrits ci-dessus pour la préparation d'un médicament destiné au traitement de pathologies humaines ou animales dans lesquelles sont impliqués les neurones silencieux, et plus particulièrement au traitement symptomatique des douleurs associées à ces pathologies.

L'invention a également pour objet l'utilisation des polyamines de formule (III) décrite ci-dessus, et plus particulièrement de la cadavérine, la putrescine, la spermidine, la spermine ou l'agmatine, ou l'utilisation des composés de formule (IV) susmentionnée, pour la préparation d'un médicament destiné au traitement des pathologies citées ci-dessus.

Parmi, les pathologies humaines ou animales susmentionnées dans lesquelles sont impliqués les neurones silencieux, susceptibles d'être traitées dans le cadre de la présente invention, on peut citer principalement les pathologies du tube digestif, de la vessie et des voies biliaires, et plus particulièrement les douleurs associées à ces pathologies, dont notamment les douleurs :
- des troubles du transit et de l'inconfort intestinal liés aux troubles fonctionnels intestinaux (dyspepsies, syndrome de l'intestin irritable, côlon irritable ...)
- des voies biliaires
- dans la rectocolite hémorragique
- dans la maladie de Crohn
- dans l'ulcère gastrique et duodénal
- dans la gastrite chronique
- dans le cancer colorectal ou gastrique
- dans la gastroentroentérite et la grippe intestinale
- liées à une pseudo-obstruction intestinale ou colique
- dans l'iléite radique
- post-opératoires digestives ou viscérales
- dans la diarrhée, spasmes, constipations, le mégacôlon, le mégarectum
- des spasmes de la vessie
- de la parésie vésicale.

Les composés selon l'invention peuvent être préparés par dissolution des différents constituants sous forme de base dans l'eau. Le sel ainsi obtenu en solution dans l'eau est ensuite précipité au moyen d'un solvant miscible à l'eau tel qu'un alcool (méthanol, éthanol, isopropanol) ou de l'acétone ou de l'acétonitrile puis collecté par filtration. La solution aqueuse peut être également lyophilisée.

Le solide obtenu dans tous les cas peut être purifié par brassage dans un solvant tel que l'éther diéthylique ou l'acétonitrile.

L'invention sera davantage illustrée à l'aide de la description détaillée qui suit de la préparation de composés de l'invention, et de leurs propriétés analgésiques mesurées sur un modèle de distension colique.

### I PROCEDES DE PREPARATION

### - Exemple 1 : cétoglutarate de mono-ornithine

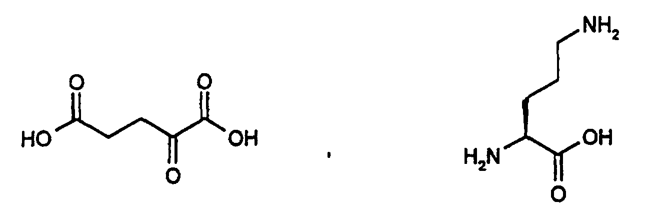

A une solution d'ornithine base dans 200 ml d'eau (1,1 N dans H₂O, 20 mmoles, 18,2 ml), ajouter l'acide α-cétoglutarique (2,92 g ; 20 mmoles). Congeler la solution et lyophiliser une nuit. Homogénéiser au mortier.

On récupère 4,71 g (85 %) d'une poudre blanche.

RMN (D₂O) : δ3,75 (t, 1H, CH(NH₂)CO₂H) ; 3,1-2,9 (m, 4H, CH₂NH₂ et CH₂COCO₂H) ; 2,7 (t, 2H, CH₂CO₂H) ; 2-1,6 (m, 4H, CH-CH₂-CH₂)

### - Exemple 2 : cétoglutarate de di-ornithine

Synthétisé de la même manière que dans le brevet 3533 M.

### - Exemple 3 : cétoglutarate de di-arginine

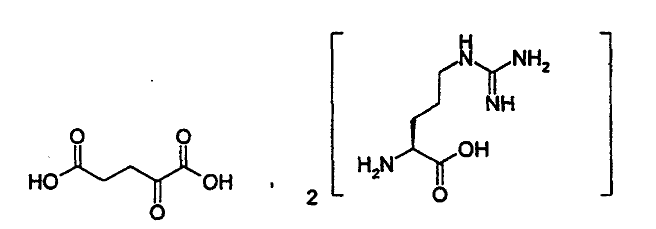

A une solution de L-arginine (10,45 g ; 60 mmoles) dans 500 ml d'eau, ajouter l'acide α-cétoglutarique (4,38 g ; 30 mmoles). Congeler la solution et lyophiliser.

On obtient un mélange sous forme de mousse et de gomme. Prélever la mousse, broyer et tamiser.

On récupère 9,26 g (62 %) de poudre blanche.

RMN (D₂O) : δ3,75 (t, 1H, CH(NH₂)CO₂H) ; 3,25 (t, 2H, -CH₂NH) ; 3 (t, 1H, CH₂COCO₂H) ; 2,45 (t, 1H, CH₂CO₂H) ; 1,9 (m, 2H, CH₂CH) ; 1,65 (m, 2H, CH₂CH₂NH)

### - Exemple 4 : cétoglutarate d'ornithine et d'arginine

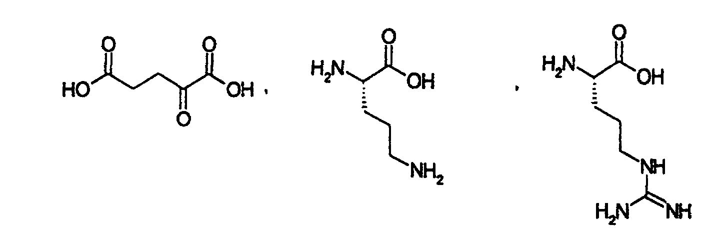

A une solution d'ornithine base (1,1 N dans H₂O ; 9,1 ml, 10 mmoles) ajouter l'acide α-cétoglutarique (1,46 g, 10 mmoles) puis la L-arginine (1,74 g, 10 mmoles). Additionner 100 ml de méthanol, on obtient une gomme qui est filtrée et reprise dans 100 ml d'éther éthylique filtrée et cristallisée dans 100 ml d'acétonitrile.

Filtrer, on récupère 1,53 g d'une poudre jaune (34 %).

RMN (D₂O) : δ3,75 (m, 2H, CH(NH₂)CO₂H ornithine et arginine) ; 3,2 (t, 2H, CH₂NH); 3 (m, 4H, CH₂NH₂ et CH₂COCO₂H) ; 2,5 (t, 2H, CH₂CO₂H) ; 2-1,5 (m, 8H, CH₂CH ornithine et arginine, CH₂CH₂NH₂, CH₂CH₂NH)

### - Exemple 5 : cétoglutarate d'ornithine et de glutamine

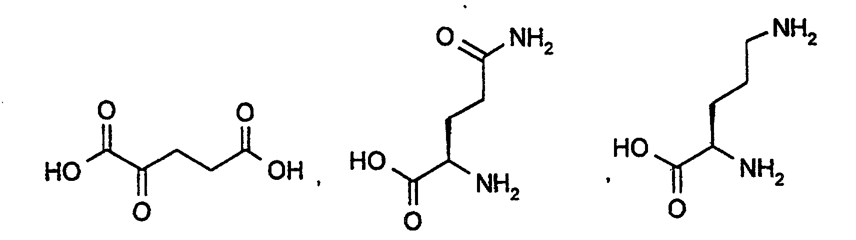

Dissoudre la glutamine (58,4 g, 0,4 moles) et l'acide α-cétoglutarique (58,4 g, 0,4 moles) dans 300 ml d'eau. Ajouter une solution d'ornithine à 40 % (117 ml, 0,4 moles). Additionner 500 ml d'éthanol puis placer cette solution à -18°C pour compléter la cristallisation.

Filtrer, laver à l'acétone et sécher sous vide.

On obtient 155 g d'un solide blanc (95 %).

RMN (D₂O) : δ3,75 (m, 2H, CH(NH₂)CO₂H ornithine et glutamine) ; 3 (m, 3,5H, CH₂NH₂ et 1,5 CH₂COCO₂H) ; 2,7 (t, 2H, CH₂CO₂H) ; 2,5 (m, 2,5H, CH₂CONH₂ et 0,5 CH₂CO₂H forme hydrate) ; 2,15 (m, 2,5H, CH₂CH glutamine et 0,5 CH₂C(OH)₂CO₂H) ; 1,9 (m, 2H, CH₂CH ornithine) ; 1,8 (m, 2H, CH₂CH₂NH₂).

### - Exemple 6 : cétobutyrate de mono-ornithine

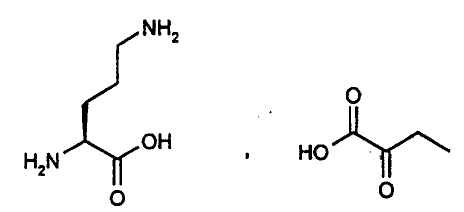

A une solution d'ornithine (1,1 N dans H₂O, 9,1 ml, 10 mmoles) ajouter l'acide α-cétoglutarique (1,02 g, 10 mmoles). Ajouter 190 ml d'acétone, glacer, filtrer, rincer à l'acétone et sécher sous vide.

On obtient 1, 07 g d'un solide blanc (46 %).

RMN (D₂O) : δ3,75 (t, 1H, CH(NH₂)CO₂H) ; 3 (t, 2H, CH₂NH₂) ; 2,75 (q, 1,5H, CH₂CH₃) ; 1,9-1,7 (m, 4,5H, CH₂CH et CH₂CH₂CH et 0,5 CH₂CH₃ forme hydrate) ; 1,1 (t, 2,5H, CH₃CH₂) ; 0,8 (t, 0,5H, CH₃CH₂ forme hydrate).

### - Exemple 7 : cétoglutarate d'ornithine de spermidine

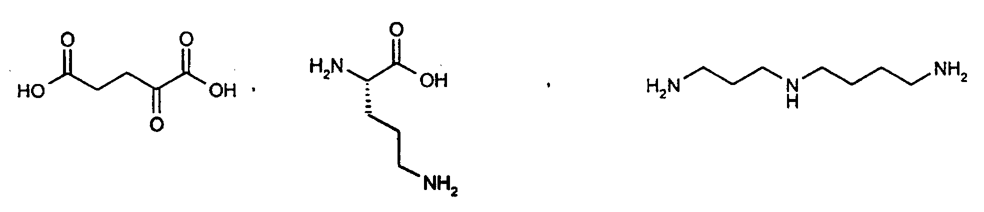

A une solution de L-ornithine base (1,1 N dans H₂O, 9,1 ml, 10 mmoles) ajouter l'acide α-cétoglutarique (1,46 g, 10 mmoles) puis la spermidine (1,45 g, 10 mmoles).

Additionner 100 ml de méthanol et 100 ml d'éther éthylique. On obtient une gomme qui est filtrée et reprise dans l'acétonitrile. On récupère 0,9 g d'un solide jaune pâle (27 %).

RMN (D₂O) : δ6,7 (m, 0,3H, CHCH₂CO₂H forme énol) ; 3,6 (t, 1H, CH(NH₂)CO₂H) ; 3 (m, 10H, CH₂NH₂ ornithine, CH₂COCO₂H, 4CH₂NH₂ et 4CH₂NH spermidine) ; 2,4 (m, 0,35H, CH₂CO₂H) ; 2,2 (m, 0,75, CH₂C(OH)₂CO₂H) ; 2-1,6(m, 11H, CH₂CH₂CH, CH₂CH₂CH₂NH, CH₂CH₂NH, CH₂CO₂H).

### - Exemple 8 : cétoglutarate de spermidine

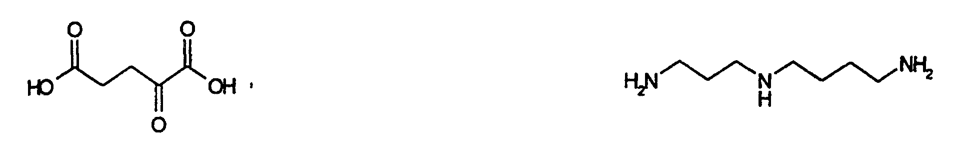

Synthétisé de façon extemporanée en mélangeant 116,3 mg (0,8 mmole) de spermidine à 117 mg (0,8 mmole) d'acide α-cétoglutarique dans 50 ml d'eau, ce qui conduit à une solution à 0,16 M.

### Exemple 9 : cétobutyrate d'arginine

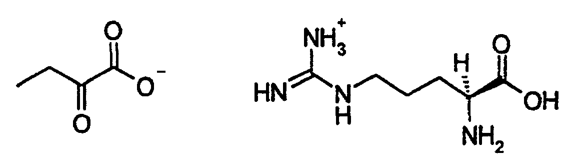

A une solution d'acide cétobutyrique (0,5 g ; 4,89 mmoles) dans H₂O (2 ml) ajouter goutte à goutte une solution d'arginine (0,84 g ; 4,89 mmoles) dans H₂O (10 ml) de telle manière que le pH reste inférieur à 7.

Ajouter 75 ml d'acétone, décanter et reprendre l'huile dans CH₃CN. Décanter et reprendre la pâte obtenue de nouveau dans l'acétonitrile. Soniquer, triturer une nuit puis filtrer.

On obtient 0,94 g (70 %) d'un solide blanc.

RMN (D₂O) : δ 3,75 (t, 1H, CH(NH₂)CO₂H) ; 3,2 (t, 2H, CH₂NH) ; 2,75 (q, 1,3 H, CH₂CH₃) ; 2-1,5 (m, 4,7 H, CH₂CH et CH₂CH₂CH et 0,7 CH₂CH₃ forme hydrate) ; 1,1 (t, 2,3 H, CH₃CH₂) ; 0,8 (t, 0,7 H, CH₃CH₂ forme hydrate).

### Exemple 10 : cétobutyrate de lysine

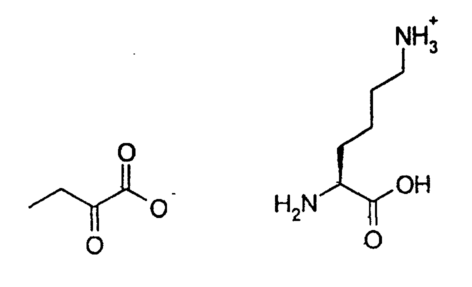

Solubiliser la lysine (1,46 g; 10 mmoles) et l'acide oxobutyrique (1,02 g; 10 mmoles) dans 5 ml de méthanol. Ajouter de l'acétone, filtrer le précipité.

On obtient 1,36 g (55 %) d'un solide blanc.

RMN (D₂O) : δ 3,7 (t, 1H, CH(NH₂) CO₂H) ; 2,95 (t, 1,7 H, CH₂NH₂) ; 2,7 (q, 1,2 H, CH₂CH₃) ; 1,9 - 1,2 (m, 6,5 H, CH₂CH et CH₂CH₂CH et CH₂CH₂CH₂CH et CH₂CH₃ forme hydrate) ; 1 (t, 1,8 H, CH₃CH₂); 0,8 (t, 0,4 H, CH₃CH₂ forme hydrate).

### Exemple 11 : cétobutyrate d'histidine

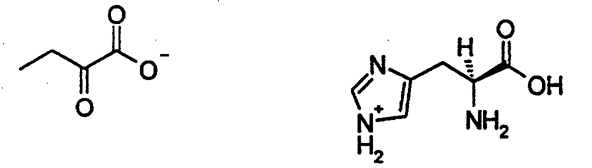

Synthétisé de la même manière que l'exemple 9.

On obtient 1,14 g (90 %) d'un solide blanc.

RMN (D₂O) : δ 8,45 (s, 1H, CH(N) NH) ; 7,3 (s, 1H, CH(C)NH) ; 4 (t, 2H, CH(NH₂)CO₂H) ; 3,3 (dd, 2H, CH₂CH) ; 2,7 (q, 1,4 H, CH₂CH₃) ; 1,7 (q, 0,15H, CH₂CH₃ forme hydrate) ; 1,05 (t, 2,2 H, CH₂CH₃) ; 0,8 (t, 0,3 H, CH₂CH₃ forme hydrate).

### Exemple 12 : α-céto-isocaproate d'arginine

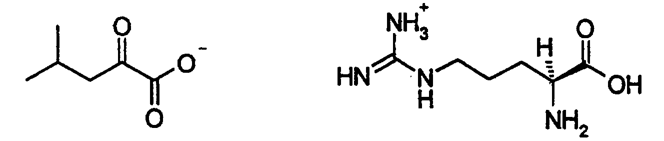

Synthétisé de la même manière que l'exemple 9.

On obtient 1,07 g (72 %) d'un solide blanc.

RMN (D₂O) : δ 3,75 (t, 1H, CH(NH₂)CO₂H) ; 3,2 (t, 2H, CH₂NH) ; 2,6 (d, 2H, CH₂CO) ; 2,05 (m, 1H, CH(CH₃)₂); 1,9 - 1,5 (m, 4H, CHCH₂CH₂) ; 0,9 (d, 6H, (CH₃)₂CH).

### Exemple 13 : α-céto-isocaproate d'ornithine

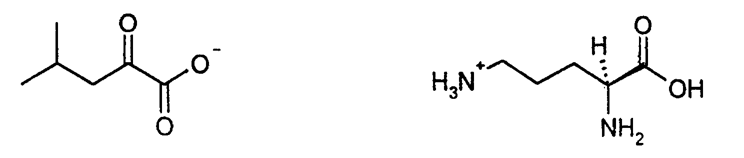

Préparer une solution d' **□-céto-isovaiérique acide (0,65 g** ; 5 mmoles) dans de l'ornithine base (5,95 ml, 111 g/l d'H₂O).Concentrer au tiers du volume et ajouter 20 ml d'acétone.

Filtrer le précipité.

On obtient 1,07 g (82 %) d'un solide blanc.

RMN (D₂O) : δ 3,75 (t, 1H, CH(NH₂)CO₂H) ; 3 (t, 2H, CH₂NH₂) ; 2,6 (d, 1H, CH₂CO) ; 2,1 (m, 1H, CHCH₂CO) ; 1,9-1,6 (m, 4H, CHCH₂CH₂) ; 0,9 (d, 6H, (CH₃)₂CH).

### Exemple 14 : α-céto -β méthylvalérate d'ornithine

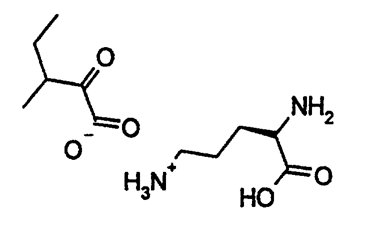

Préparer une solution d'acide α-céto -β méthylvalérique (0,65 g ; 5 mmoles) et d'ornithine base (5,95 ml, 111 g/l H₂O).

Concentrer au tiers du volume et ajouter 60 ml d'acétone.

Filtrer le précipité.

On obtient 1,03 g (78 %) d'un solide blanc.

RMN (D₂O) : δ 3,75 (t, 1H, CH(NH₂)CO₂H) ; 3,05 (t, 2H, CH₂NH₂) ; 2,9 (m, CHCO) ; 1,9 (m, 2H, CHCH₂) ; 1,85 - 1,4 (m, 4H, CH₂CH₂NH₂ et CH₃CH₂) ; 1,1 (d, 3H, CH₃CH) ; 0,9 (t, 3H, CH₃CH).

### Exemple 15 : α-céto -β méthylvalérate d'arginine

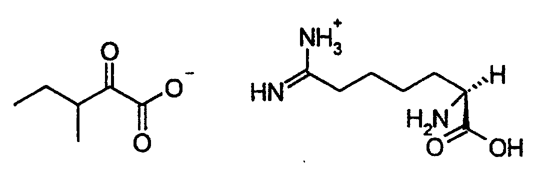

Synthétisé de la même manière que l'exemple 9.

RMN (D₂O) : δ 3,7 (t, 1H, CH(NH₂)CO2H) ; 3,2 (t, 2H, CH₂NH) ; 2,9 (m, 1H, CHCO) ; 1,9 (m, 2H, CHCH₂) ; 1,7-1,4(m, 4H, CH₂CH₂NH₂ et CH₃CH₂); 1,1(d, 3H, CH₃CH) ; 0,9 (t, 3H, CH₃CH₂).

### Exemple 16 : α-céto-isovalérate d'arginine

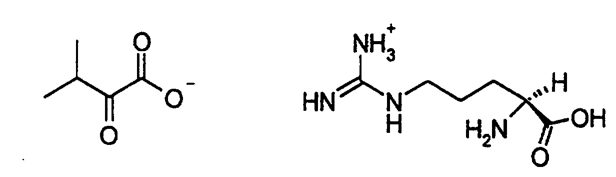

Synthétisé de la même manière que l'exemple 9.

RMN (D₂O) : δ 3,7 (t, 1H, CH(NH₂)CO2H); 3,2 (t, 2H, CH₂NH); 3(m, 0,9 H, CH(CH₃)₂); 1,9 - 1,5 (m, 4,1H, CH₂CH₂CH et 0,1 CH(CH₃) forme hydrate); 1,1 (d, 5,7H, CH(CH₃)₂); 0,85 (d, 0,3H, CH(CH₃)₂ forme hydrate).

### Exemple 17 : α-céto-isovalérate d'ornithine

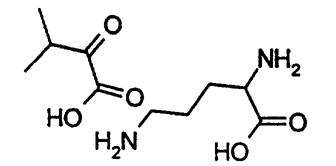

Synthétisé de la même manière que l'exemple 14.

RMN (D₂O) : δ 3,7 (t, 1H, CH(NH₂)CO₂H) ; 3 (m, 3H, CH₂NH₂ et CHCO) ; 1,95-1,6 (m, 4H, CH₂CH₂CH) ; 1,1 (d, 6H, CH(CH₃)₂).

### II PHARMACOLOGIE

### - Distension colique

L'activité analgésique a été étudiée sur un modèle de douleur digestive chez le rat vigile. Cette douleur est provoquée par la distension du côlon à l'aide d'un ballonnet.

### - Protocole

Les rats mâles Sprague-Dawley d'environ 180 g à jeun depuis la veille sont utilisés. Sous légère anesthésie au fluothane, une sonde intrarectale est introduite à 5 cm de l'anus et 1,5 ml d'acide acétique à 1 % est injecté. Une heure trente après l'irritation, un ballonnet en latex (∅ à vide 2 mm, longueur 1 cm) monté sur un cathéter en polyéthylène est introduit dans le côlon sur le site de l'irritation.

Le produit à tester ou le véhicule (eau distillée) est administré per os sous un volume de 1 ml, puis le rat est mis en observation dans un cristallisoir.

La distension du côlon est réalisée 2h30 après l'irritation. Elle est effectuée sous un volume fixe égale à 1,5 ml d'eau distillée. La distension colique provoque une douleur digestive objectivée par des crampes abdominales dont le nombre reflète l'intensité de la douleur. La distension colique est maintenue pendant 10 minutes au cours desquelles les crampes abdominales sont dénombrées.

L'analyse statistique est effectuée à l'aide du test de Dunnett qui compare un même groupe d'animaux véhicule (40) à plusieurs groupes de rats (6 animaux par groupe) ayant reçu les molécules étudiées. Le seuil de signification est fixé à 5 %.

Les molécules sont testées par voie orale à 1-10-20 mg/kg. Elles sont solubilisées dans de l'eau distillée.

Le véhicule utilisé comme placebo se compose d'eau distillée.

Les comparateurs 1 à 5 sont des produits disponibles dans le commerce et sont utilisés tels que, sauf le chlorhydrate d'ornithine qui est relargué par des méthodes connues dans la littérature et stockée en solution dans l'eau.

### - Résultats

Vingt à vingt deux crampes abdominales, en moyenne, sont observées pendant les 10 minutes de distension colique chez le groupe de rats véhicule (n = 40 rats).

Les molécules testées diminuent significativement (p < 0,05) la douleur digestive lorsque le nombre de crampes abdominales est inférieur ou égal à 15.

Les résultats des composés selon l'invention sont donnés ci-après (tableau I).

Les composés selon l'invention diminuent significativement la douleur digestive.

**TABLEAU I**

| Distension colique chez le rat à 1, 10 et 20 mg/kg p.o. (nombre moyen de crampes abdominales ± écart-type) | | | | |
|---|---|---|---|---|
| COMPOSE | PLACEBO (véhicule) | 1 mg/kg | 10 mg/kg | 20 mg/kg |
| Acide α-cétoglutarique Comparateur 1 | 21 ± 2 | 17 ± 1 | 17 ± 4 | 22 ± 6 |
| Ornithine, HCl Comparateur 2 | 22 ± 4 | 20 ± 3 | 19 ± 6 | 18 ± 6 |
| Acide α-cétobutyrique Comparateur 3 | 21 ± 1 | 19 ± 2 | 20 ± 3 | 19 ± 3 |
| Glutamine Comparateur 4 | 23 ± 5 | 21 ± 2 | 13* ± 4 | 8* ± 3 |
| Arginine Comparateur 5 | 20 ± 1 | 20 ± 6 | 20 ± 9 | 13* ± 6 |
| Cétoglutarate de mono-ornithine Exemple 1 | 20 ± 2 | 11* ± 7 | 11* ± 9 | 15* ± 7 |
| Cétoglutarate de di-ornithine Exemple 2 | 23 ± 5 | 14* ± 2 | 9* ± 4 | 8* ± 3 |
| Cétoglutarate de di-arginine Exemple 3 | 26 ± 9 | 17 ± 9 | 12* ± 4 | 10* ± 5 |
| Cétoglutarate d'ornithine d'arginine Exemple 4 | 19 ± 1 | 18 ± 2 | 14* ± 9 | 13* ± 5 |
| Cétoglutarate d'ornithine de glutamine Exemple 5 | 23 ± 5 | 11* ± 4 | 12* ± 5 | 11* ± 6 |
| Cétobutyrate d'ornithine Exemple 6 | 22 ± 1 | 12* ± 5 | 14* ± 7 | 8* ± 3 |
| Cétoglutarate d'ornithine de spermidine Exemple 7 | 21 ± 2 | 13* ± 2 | 14* ± 3 | 9* ± 4 |
| Cétoglutarate de spermidine Exemple 8 | 18 ± 0,7 | 8* ± 4 | 9* ± 4 | 8* ± 2 |

| | | | | |
|---|---|---|---|---|
| * Statistiquement différent du placebo à p <0,05 (test de Dunnett) | | | | |

## Revendications

1. Utilisation de composés de formule générale (I) suivante :
(X)ₙ₁,Y,(Z)ₙ₂ (I)
dans laquelle :
- n₁ et n₂ représentent 0 ou 1, sous réserve que lorsque n₁ = 0 alors n₂ = 1, et que lorsque n₂ = 0 alors n₁ = 1,
- X représente un aminoacide naturel, sous réserve que lorsque n₂ = 0 alors X représente un aminoacide basique,
- Y représente un cétoacide de formule (II) suivante :
R-CO-COOH (II)
dans laquelle R représente un groupe alkyle ou acide alcanoïque, substitué ou non de 1 à 10 atomes de carbone,
- Z représente :
• un aminoacide naturel,
• ou, une polyamine comprenant au moins deux fonctions amines primaires, secondaires ou tertiaires, espacée par une chaîne hydrocarbonée linéaire ou ramifiée de 3 à 10 atomes de carbone,
pour la préparation d'un médicament destiné au traitement de pathologies humaines ou animales dans lesquelles sont impliqués les neurones silencieux, telles que les pathologies du tube digestif, de la vessie et des voies biliaires.

2. Utilisation selon la revendication 1, de composés de formule (I) dans laquelle :
- X représente un acide aminé choisi parmi l'ornithine, l'arginine ou la glutamine,
- Y représente un cétoacide de formule (II) suivante :
R-CO-COOH (II)
dans laquelle lorsque R représente :
• -CH₃, ledit cétoacide est l'acide pyruvique,
• -CH₂-CH₃, ledit cétoacide est l'acide α-cétobutyrique,
• -CH(CH₃)₂, ledit cétoacide est l'acide α-cétoisovalérique,
• -CH(CH₃)-CH₂-CH₃, ledit cétoacide est l'acide α-céto β-méthylvalérique,
• -CH₂-CH(CH₃)₂, ledit cétoacide est l'acide α-cétoisocaproïque,
• -(CH₂)₂-COOH, ledit cétoacide est l'acide α-cétoglutarique,
• -(CH₂)₃-COOH, ledit cétoacide est l'acide α-cétoadipique,
- Z représente :
• un aminoacide choisi parmi l'ornithine, l'arginine, la lysine, l'histidine, ou la glutamine,
• ou, une polyamine de formule (III) suivante :
R₁-HN-(CH₂)n-NH-R₂ (III)
dans laquelle :
. n représente un nombre entier compris entre 4 et 5, et
* lorsque n = 4,
.. R₁ et R₂ représentent H,
.. ou, R₁ représente H et R₂ représente (CH₂)₃NH₂,
.. ou, R₁ et R₂ représentent (CH₂)₃NH₂,
* lorsque n = 5,
.. R₁ et R₂ représentent H,
.. ou, R₁ représente H et R₂ représente un groupe de formule

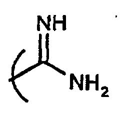

3. Utilisation selon la revendication 1 ou 2, de composés de formule (I) dans laquelle :
- n₁ = 1, et n₂ = 0 ou 1,
- X représente un acide aminé choisi parmi l'ornithine, l'arginine ou la glutamine,
- Y représente un cétoacide choisi parmi l'acide α-cétoglutarique, ou l'acide α-cétobutyrique,
- et, lorsque n₂ = 1, Z représente :
. un acide aminé choisi parmi l'ornithine, l'arginine ou la glutamine,
. ou une polyamine de formule (III) choisie parmi la cadavérine, la putrescine, la spermidine, la spermine ou l'agmatine.

4. Utilisation selon l'une des revendications 1 à 3, de composés de formule (I) dans laquelle :
- n₁ = 1, et n₂ = 0,
- X représente l'ornithine et Y représente l'acide α-cétoglutarique, à savoir l'α-cétoglutarate de mono-ornithine,
- X représente l'ornithine et Y représente l'acide α-cétobutyrique, à savoir l'α-cétobutyrate de mono-ornithine,
- X représente l'arginine et Y représente l'acide α-cétobutyrique, à savoir l'α-cétobutyrate d'arginine,
- X représente la lysine et Y représente l'acide α-cétobutyrique, à savoir l'α-cétobutyrate de lysine,
- X représente l'histidine et Y représente l'acide α-cétobutyrique, à savoir l'α-cétobutyrate d'histidine,
- X représente l'arginine et Y représente l'acide α-céto-isocaproïque, à savoir l'α-céto-isocaproate d'arginine,
- X représente l'ornithine et Y représente l'acide α-céto-isocaproïque, à savoir l'α-céto-isocaproate d'ornithine,
- X représente l'ornithine et Y représente l'acide α-céto-β méthylvalérique, à savoir l'α-céto-β méthylvalérate d'ornithine,
- X représente l'arginine et Y représente l'acide α-céto-β méthylvalérique, à savoir l'α-céto-β méthylvalérate d'arginine,
- X représente l'arginine et Y représente l'acide α-céto-isovalérique, à savoir l'α-céto-isovalérate d'arginine,
- X représente l'ornithine et Y représente l'acide α-céto-isovalérique, à savoir l'α-céto-isovalérate d'ornithine.

5. Utilisation selon l'une des revendications 1 à 3, de composés de formule (I) dans laquelle :
- n₁ = 1, et n₂ = 1,
- X représente l'ornithine, Y représente l'acide α-cétoglutarique, et Z représente l'ornithine, à savoir l'α-cétoglutarate de di-ornithine,
- X représente l'arginine, Y représente l'acide α-cétoglutarique, et Z représente l'arginine, à savoir l'α-cétoglutarate de di-arginine,
- X représente l'ornithine, Y représente l'acide α-cétoglutarique, et Z représente la glutamine, à savoir l'α-cétoglutarate d'ornithine et de glutamine,
- X représente l'ornithine, Y représente l'acide α-cétoglutarique, et Z représente la spermidine, à savoir l'α-cétoglutarate d'ornithine et de spermidine.

6. Utilisation selon l'une des revendications 1 à 3, de l'α-cétoglutarate de diornithine.

7. Utilisation selon la revendication 1 ou 2, de composés de formule (I) dans laquelle :
- n₁ = 0 ou 1, et n₂ = 1,
- lorsque n₁ = 1, X représente un aminoacide naturel,
- Y représente un cétoacide choisi parmi l'acide α-cétoglutarique, ou l'acide α-cétobutyrique,
- Z représente une polyamine de formule (III).

8. Utilisation selon la revendication 7, de composés de formule (I) dans laquelle :
- lorsque n₁ = 1, X représente l'ornithine, l'arginine ou la glutamine,
- Z représente la cadavérine, la putrescine, la spermidine, la spermine ou l'agmatine.

9. Utilisation selon la revendication 7 ou 8, du composé de formule (I) dans laquelle n₁ = 0, et n₂ = 1, Y représente l'acide α-cétoglutarique et Z représente la spermidine, à savoir l'α-cétoglutarate de spermidine

10. Utilisation selon l'une des revendications 1 à 9, de composés se présentant sous forme de sels entre deux constituants X et Y, ou Y et Z, ou entre les trois constituants X, Y et Z.

11. Utilisation selon l'une des revendications 1 à 10, de composés de formule (I) au sein desquels la proportion en poids des différents constituants est comprise entre 0,8 et 1,2, de sorte que la somme des proportions de chacun des constituants est égale à 2 dans un sel entre deux constituants, ou est égale à 3 dans un sel entre deux constituants.

12. Utilisation selon l'une des revendications 1 à 11, de composés de formule (I) au sein desquels les différents constituants X et Y, ou Y et Z, ou X, Y et Z sont dans un rapport équimolaire, c'est-à-dire que chacun des constituants est dans une proportion en poids de 1, de sorte que la somme des proportions de chacun des constituants est égale à 2 dans un sel entre deux constituants X et Y, ou Y et Z, ou est égale à 3 dans un sel entre les trois constituants X, Y et Z.

13. Composés de formule générale (IV) suivante :
(X)ₙₐ,(Y)_{nb},Z (IV)
dans laquelle :
- nₐ et n_{b} représentent 0 ou 1, sous réserve que lorsque nₐ = 0 alors n_{b} = 1, et que lorsque n_{b} = 0 alors nₐ = 1,
- X représente un aminoacide naturel,
- Y représente un cétoacide de formule (II) suivante :
R-CO-COOH (II)
dans laquelle R représente un groupe alkyle ou acide alcanoïque, substitué ou non, de 1 à 10 atomes de carbone,
- Z représente une polyamine comprenant au moins deux fonctions amines primaires, secondaires ou tertiaires, espacée par une chaîne hydrocarbonée linéaire ou ramifiée de 3 à 10 atomes de carbone.

14. Composé selon la revendication 13, de formule générale (IV) dans laquelle
- X représente un aminoacide basique choisi parmi l'ornithine, l'arginine, la lysine, ou l'histidine,
- Y représente un cétoacide de formule (II) suivante :
R-CO-COOH (II)
dans laquelle lorsque R représente :
• -CH₃, ledit cétoacide est l'acide pyruvique,
• -CH₂-CH₃, ledit cétoacide est l'acide α-cétobutyrique,
• -CH(CH₃)₂, ledit cétoacide est l'acide α-cétoisovalérique,
• -CH(CH₃)-CH₂-CH₃, ledit cétoacide est l'acide α-céto β-méthylvalérique,
• -CH₂-CH(CH₃)₂, ledit cétoacide est l'acide α-cétoisocaproïque,
• -(CH₂)₂-COOH, ledit cétoacide est l'acide α-cétoglutarique,
• -(CH₂)₃-COOH, ledit cétoacide est l'acide α-cétoadipique,
- Z représente une polyamine de formule (III) suivante :
R₁-HN-(CH₂)n-NH-R₂ (III)
dans laquelle :
. n représente un nombre entier compris entre 4 et 5, et
* lorsque n = 4,
.. R₁ et R₂ représentent H,
.. ou, R₁ représente H et R₂ représente (CH₂)₃NH₂,
.. ou, R₁ et R₂ représentent (CH₂)₃NH₂,
* lorsque n = 5,
.. R₁ et R₂ représentent H,
.. ou, R₁ représente H et R₂ représente un groupe de formule

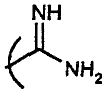

15. Composé selon la revendication 13 ou 14, de formule générale (IV) dans laquelle Z représente une polyamine choisie parmi la cadavérine, la putrescine, la spermidine, la spermine ou l'agmatine.

16. Composé selon l'une des revendications 13 à 15, de formule générale (IV) dans laquelle nₐ = 0 et n_{b} = 1, Y représente l'acide α-cétoglutarique, et Z représente la spermidine, à savoir l'α-cétoglutarate de spermidine.

17. Composition pharmaceutique **caractérisée en ce qu'**elle comprend un composé selon l'une des revendications 13 à 16, en association avec un véhicule pharmaceutiquement acceptable.

## Patentansprüche

1. Verwendung von Verbindungen der folgenden allgemeinen Formel (I):
(X)_{n₁} , Y, (Z)_{n₂} (I)
in welcher:
- n₁ und n₂ 0 oder 1 darstellen, unter dem Vorbehalt, dass wenn n₁ = 0 dann n₂ = 1, und, dass, wenn n₂ = 0 dann n₁ = 1,
- X eine natürliche Aminosäure darstellt, unter dem Vorbehalt, dass, wenn n₂ = 0 dann X eine basische Aminosäure darstellt,
- Y eine Ketosäure der folgenden Formel (II) darstellt:
R-CO-COOH (II)
in welcher R eine Alkylgruppe oder Alkansäure darstellt, die mit 1 bis 10 Kohlenstoffatomen substituiert ist oder nicht,
- Z darstellt:
• eine natürliche Aminosäure,
• oder ein wenigstens 2 primäre, sekundäre oder tertiäre Aminfunktionen umfassendes Polyamin, das von einer linearen oder verzweigten Kohlenstoffkette mit 3 bis 10 Kohlenstoffatomen als Platzhalter gehalten wird,
zur Herstellung eines Medikaments, das zur Behandlung von menschlichen oder tierischen Erkrankungen bestimmt ist, bei welchen stille Neuronen beteiligt sind, wie etwa Erkrankungen des Verdauungstraktes, der Blase und der Gallenwege.

2. Verwendung gemäß Anspruch 1 von Verbindungen der Formel (I) in welcher:
- X eine Aminosäure, ausgewählt aus Ornithin, Arginin oder Glutamin darstellt,
- Y eine Ketosäure der folgenden Formel (II) darstellt:
R-CO-COOH (II)
in welcher, wenn R darstellt:
• -CH₃, die Ketosäure Brenztraubensäure ist,
• -CH₂-CH₃, die Ketosäure α-Ketobutansäure ist,
• -CH(CH₃)₂, die Ketosäure α-Ketoisovaleriansäure ist,
• -CH(CH₃)-CH₂-CH₃, die Ketosäure α-Keto-β-methylvaleriansäure ist,
• -CH₂-CH(CH₃)₂, die Ketosäure α-Ketoisocaproinsäure ist,
• -(CH₂)₂-COOH, die Ketosäure α-Ketoglutarsäure ist,
• -(CH₂)₃-COOH, die Ketosäure α-Ketoadipinsäure ist,
- Z darstellt:
• eine Aminosäure, ausgewählt aus Ornithin, Arginin, Lysin, Histidin oder Glutamin,
• oder ein Polyamin der folgenden Formel (III):
R₁-HN-(CH₂)n-NH-R₂ (III)
in welcher:
. n eine ganze Zahl, umfasst zwischen 4 und 5 darstellt, und
* wenn n = 4,
.. R₁ und R₂ H darstellen,
.. oder, R₁ H darstellt und R₂ (CH₂)₃NH₂ darstellt,
.. oder, R₁ und R₂ (CH₂)₃NH₂ darstellen,
* wenn n = 5,
.. R₁ und R₂ H darstellen,
.. oder, R₁ H darstellt und R₂ eine Gruppe der folgenden Formel darstellt:

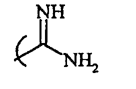

3. Verwendung gemäß Anspruch 1 oder 2, von Verbindungen der Formel (I) in welcher:
- n₁ = 1, und n₂ = 0 oder 1,
- X eine Aminosäure, ausgewählt aus Ornithin, Arginin oder Glutamin darstellt,
- Y eine Ketosäure ausgewählt aus α-Ketoglutarsäure, oder α-Ketobutansäure darstellt,
- und wenn n₂ = 1, Z darstellt:
• eine Aminosäure, ausgewählt aus Ornithin, Arginin oder Glutamin,
• oder ein Polyamin der Formel (III), ausgewählt aus Cadaverin, Putrescin, Spermidin, Spermin oder Agmatin.

4. Verwendung gemäß einem der Ansprüche 1 bis 3, von Verbindungen der Formel (I), in welcher:
- n₁ = 1, und n₂ = 0,
- X Ornithin darstellt und Y α-Ketoglutarsäure darstellt, nämlich das α-Ketoglutarat von Mono-Ornithin,
- X Ornithin darstellt und Y α-Ketobutansäure darstellt, nämlich das α-Ketobutyrat von Mono-Ornithin,
- X Arginin darstellt und Y α-Ketobutansäure darstellt, nämlich das α-Ketobutyrat von Arginin,
- X Lysin darstellt und Y α-Ketobutansäure darstellt, nämlich das α-Ketobutyrat von Lysin,
- X Histidin darstellt und Y α-Ketobutansäure darstellt, nämlich das α-Ketobutyrat von Histidin,
- X Arginin darstellt und Y α-Ketoisocaproinsäure darstellt, nämlich α-Ketoisocaproat von Arginin,
- X Ornithin darstellt und Y α-Ketoisocaproinsäure darstellt, nämlich das α-Ketoisocaproat von Ornithin,
- X Ornithin darstellt und Y α-Keto-β-methylvaleriansäure darstellt, nämlich das α-Keto-β- methylvalerat von Ornithin,
- X Arginin darstellt und Y α-Keto-β-methylvaleriansäure darstellt, nämlich das α-Keto-β-methylvalerat von Arginin,
- X Arginin darstellt und Y α-Ketoisovaleriansäure darstellt, nämlich das α-Ketoisovalerat von Arginin,
- X Ornithin darstellt und Y α-Ketoisovaleriansäure darstellt, nämlich das α-Ketoisovalerat von Ornithin.

5. Verwendung gemäß einem der Ansprüche 1 bis 3, von Verbindungen der Formel (I), in welcher:
- n₁ = 1, und n₂ = 1,
- X Ornithin darstellt, Y α-Ketoglutarsäure darstellt, und Z Ornithin darstellt, nämlich das α-Ketoglutarat von Diornithin,
- X Arginin darstellt, Y α-Ketoglutarsäure darstellt, und Z Arginin darstellt, nämlich das α-Ketoglutarat von Diarginin,
- X Ornithin darstellt, Y α-Ketoglutarsäure darstellt und Z Glutamin darstellt, nämlich das α-Ketoglutarat von Ornithin und Glutamin,
- X Ornithin darstellt, Y α-Ketoglutarsäure darstellt und Z Spermidin darstellt, nämlich das α-Ketoglutarat von Ornithin und Spermidin.

6. Verwendung gemäß einem der Ansprüche 1 bis 3, von dem α-Ketoglutarat von Diornithin.

7. Verwendung gemäß Anspruch 1 oder 2, von Verbindungen der Formel (I), in welcher:
- n₁ = 0 oder 1, und n₂ = 1,
- wenn n₁ = 1, Y eine natürliche Aminosäure darstellt,
- Y eine Ketosäure darstellt, ausgewählt aus α-Ketoglutarsäure, oder α-Ketobutansäure,
- Z ein Polyamin der Formel (III) darstellt.

8. Verwendung gemäß Anspruch 7, von Verbindungen der Formel (I), in welcher:
- wenn n₁ = 1, Y Ornithin, Arginin oder Glutamin darstellt,
- Z Cadaverin, Putrescin, Spermidin, Spermin oder Agmatin darstellt.

9. Verwendung gemäß Anspruch 7 oder 8, von der Verbindung der Formel (I), in welcher n₁ = 0 und n₂ = 1, Y α-Ketoglutarsäure darstellt und Z Spermidin darstellt, nämlich das α-Ketoglutarat von Spermidin.

10. Verwendung gemäß einem der Ansprüche 1 bis 9, von Verbindungen, die sich in der Form von Salzen zwischen zwei Konstituenten X und Y, oder Y und Z oder zwischen drei Konstituenten X, Y und Z präsentieren.

11. Verwendung gemäß einem der Ansprüche 1 bis 10, von Verbindungen der Formel (I), innerhalb welcher das Gewichtsverhältnis von verschiedenen Konstituenten zwischen 0,8 und 1,2 umfasst ist, so dass die Summe der Verhältnisse von jedem der Konstituenten in einem Salz zwischen zwei Konstituenten 2 entspricht, oder in einem Salz zwischen zwei Konstituenten 3 entspricht.

12. Verwendung gemäß einem der Ansprüche 1 bis 11, von Verbindungen der Formel (I), bei welchen die verschiedenen Konstituenten X und Y, oder Y und Z, oder X, Y und Z in einem equimolaren Verhältnis sind, das heißt, das jeder der Konstituenten in einem Gewichtsverhältnis von 1 ist, so dass die Summe der Verhältnisse von jedem der Konstituenten entspricht in einem Salz zwischen zwei Konstituenten X und Y oder Y und Z 2, oder in einem Salz zwischen drei Konstituenten X, Y und Z 3 entspricht.

13. Verbindungen der allgemeinen folgenden Formel (IV):
(X)_{nₐ},(Y)_{n_{b}},Z (IV)
in welcher:
- nₐ und n_{b} 0 oder 1 darstellen, unter dem Vorbehalt, dass wenn nₐ = 0 dann n_{b} = 1, und, dass wenn n_{b} = 0, dann nₐ = 1,
- X einen natürliche Aminosäure darstellt,
- Y eine Ketosäure der folgenden Formel (II) darstellt:
R-CO-COOH (II)
in welcher R eine Alkylgruppe oder Alkansäure darstellt, die mit 1 bis 10 Kohlenstoffatomen substituiert ist oder nicht,
- Z ein wenigstens zwei primäre, sekundäre oder tertiäre Aminfunktionen umfassendes Polyamin darstellt, das von einer linearen oder verzweigten Kohlenwasserstoffkette mit 3 bis 10 Kohlenstoffatomen als Platzhalter gehalten wird.

14. Verbindung gemäß Anspruch 13, der allgemeinen Formel (IV) in welcher
- X eine Aminosäure, ausgewählt aus Ornithin, Arginin oder Glutamin darstellt,
- Y eine Ketosäure der folgenden Formel (II) darstellt:
R-CO-COOH (II)
in welcher, wenn R darstellt:
• -CH₃, die Ketosäure Brenztraubensäure ist,
• -CH₂-CH₃, die Ketosäure α-Ketobutansäure ist,
• -CH(CH₃)₂, die Ketosäure α-Ketoisovaleriansäure ist,
• -CH(CH₃)-CH₂-CH₃, die Ketosäure α-Keto-β-methylvaleriansäure ist,
• -CH₂-CH(CH₃)₂, die Ketosäure α-Ketoisocaproinsäure ist,
• -(CH₂)₂-COOH, die Ketosäure α-Ketoglutarsäure ist,
• -(CH₂)₃-COOH, die Ketosäure α-Ketoadipinsäure ist,
- Z darstellt ein Polyamin der folgenden Formel (III):
R₁-HN-(CH₂)n-NH-R₂ (III)
in welcher:
. n eine ganze Zahl umfasst zwischen 4 und 5 darstellt, und
* wenn n = 4,
.. R₁ und R₂ H darstellen,
.. oder, R₁ H darstellt und R₂ (CH₂)₃NH₂ darstellt,
.. oder, R₁ und R₂ (CH₂)₃NH₂ darstellen,
* wenn n = 5,
.. R₁ und R₂ H darstellen,
.. oder, R₁ H darstellt und R₂ eine Gruppe der folgenden Formel darstellt:

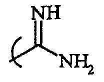

15. Verbindung gemäß Anspruch 13 oder 14, der allgemeinen Formel (IV), in welcher Z eine Polyamin darstellt, ausgewählt aus Cadaverin, Putrescin, Spermidin, Spermin oder Agmatin.

16. Verbindung gemäß einem der Ansprüche 13 bis 15, der allgemeinen Formel (IV), in welcher nₐ = 0 und n_{b} = 1, Y α-Ketoglutarsäure darstellt, und Z Spermidin darstellt, nämlich das α-Ketoglutarat von Spermidin.

17. Pharmazeutische Zusammensetzung, **dadurch gekennzeichnet, dass** sie eine Verbindung gemäß einem der Ansprüche 13 bis 16 umfasst, in Verbindung mit einem pharmazeutisch akzeptablen Träger.

## Claims

1. Use of compounds of the following general formula (I):
(X)_{n₁}, Y, (Z)_{n₂} (I)
in which:
- n₁ and n₂ represent 0 or 1, provided that when n₁ = 0 then n₂ = 1, and when n₂ = 0 then n₁ = 1,
- X represents a natural amino acid, provided that when n₂ = 0 then X is a basic amino acid,
- Y represents a keto acid of the following formula (II):
R-CO-COOH (II)
in which R represents an alkyl or alcanoic acid group, substituted or not, of 1 to 10 carbon atoms,
- Z represents:
• a natural amino acid,
• or, a polyamine comprising at least two primary, secondary or tertiary amine functions, spaced by a linear or branched hydrocarbon chain of 3 to 10 carbon atoms,
for the preparation of a medication adapted for the treatment of human or animal pathologies in which the silent neurons are involved, such as pathologies of the digestive tract, the bladder or the biliary ducts.

2. Use according to claim 1, of compounds of formula (I), in which:
- X represents an amino acid, selected from ornithine, arginine, or glutamine,
- Y represents a keto acid of the following formula (II):
R-CO-COOH (II)
in which when R represents:
• -CH₃, said keto acid is pyruvic acid,
• -CH₂-CH₃, said keto acid is α-keto-butyric acid,
• -CH(CH₃)₂, said keto acid is α-keto-isovaleric acid,
• -CH(CH₃)-CH₂-CH₃, said keto acid is α-keto β-methylvaleric acid,
• -CH₂-CH(CH₃)₂, said keto acid is α-keto isocaproic acid,
• -(CH₂)₂-COOH, said keto acid is α-keto glutaric acid,
• -(CH₂)₃-COOH, said keto acid is α-keto adipic acid,
- Z represents:
• an amino acid selected from ornithine, arginine, lysine, histidine or glutamine,
• or a polyamine of the following formula (III):
R₁-HN-(CH₂)ₙ-NH-R₂ (III)
in which:
. n represents a whole number comprised between 4 and 5, and
* when n = 4,
.. R₁ and R₂ represent H,
.. or, R₁ represents H and R₂ represents (CH₂)₃NH₂,
.. or, R₁ and R₂ represent (CH₂)₃NH₂,
* when n = 5,
.. R₁ and R₂ represent H,
.. or, R₁ represents H and R₂ represents a group of the formula

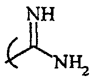

3. Use according to claim 1 or 2, of compounds of formula (I) in which:
- n₁ = 1, and n₂ = 0 or 1,
- X represents an amino acid selected from ornithine, arginine or glutamine,
- Y represents a keto acid selected from α-keto glutaric acid or α-keto-butyric acid,
- and, when n₂ = 1, Z represents:
• an amino acid, selected from ornithine, arginine or glutamine,
• or a polyamine of formula (III) selected from cadaverine, putrescine, spermidine, spermine or agmatine.

4. Use according to one of claims 1 to 3, of compounds of formula (I) in which:
- n₁ = 1, and n₂ = 0,
- X represents ornithine and Y represents α-keto glutaric acid, namely mono-ornithine α-keto glutarate,
- X represents ornithine and Y represents α-ketobutyric acid, namely mono-ornithine α-ketobutyrate,
- X represents arginine and Y represents α-ketobutyric acid, namely arginine α-ketobutyrate,
- X represents lysine and Y represents α-ketobutyric acid, namely lysine α-ketobutyrate,
- X represents histidine and Y represents α-ketobutyric acid, namely histidine α-ketobutyrate,
- X represents arginine and Y represents α-ketoisocaproic acid, namely arginine α-ketoisocaproate,
- X represents ornithine and Y represents α-ketoisocaproic acid, namely ornithine α-ketoisocaproate,
- X represents ornithine and Y represents a-keto-β-methylvaleric acid, namely ornithine α-keto-β-methylvalerate,
- X represents arginine and Y represents α-keto-β-methylvaleric acid, namely arginine α-keto-β-methylvalerate,
- X represents arginine and Y represents α-keto-isovaleric acid, namely arginine α-keto-isovalerate,
- X represents ornithine and Y represents α-keto-isovaleric acid, namely ornithine α-keto-isovalerate.

5. Use according to one of claims 1 to 3, of compounds of formula (I) in which:
- n₁ = 1, and n₂ = 1,
- X represents ornithine, Y represents α-keto-glutaric acid, and Z represents ornithine, namely diornithine α-keto-glutarate,
- X represents arginine, Y represents α-keto-glutaric acid, and Z represents arginine, namely diarginine α-keto-glutarate,
- X represents ornithine, Y represents α-keto-glutaric acid, and Z represents glutamine, namely ornithine and glutamine α-keto-glutarate,
- X represents ornithine, Y represents α-keto-glutaric acid, and Z represents spermidine, namely ornithine and spermidine α-keto-glutarate.

6. Use according to one of claims 1 to 3, of diornithine α-keto-glutarate.

7. Use according to claim 1 or 2, of compounds of formula (I) in which:
- n₁ = 0 or 1, and n₂ = 1,
- when n₁ = 1, X represents a natural amino acid,
- Y represents a keto acid selected from α-keto glutaric acid or α-keto-butyric acid,
- Z represents a polyamine of formula (III).

8. Use according to claim 7, of compounds of formula (I) in which:
- when n₁ = 1, X represents ornithine, arginine or glutamine,
- Z represents cadaverine, putrescine, spermidine, spermine or agmatine.

9. Use according to claim 7 or 8, of the compound of formula (I) in which n₁ = 0 and n₂ = 1, Y represents α-keto-glutaric acid and Z represents spermidine, namely spermidine α-keto-gluterate.

10. Use according to one of claims 1 to 9, of compounds in the form of salts between two constituents X and Y, or Y and Z, or between the three constituents X, Y and Z.

11. Use according to one of claims 1 to 10, of compounds of formula (I) in which the weight proportion of the different constituents is comprised between 0,8 and 1,2, such that the sum of the proportions of each of the constituents is equal to 2 in a salt between two constituents, or is equal to 3 in a salt between two constituents.

12. Use according to one of claims 1 to 11, of compounds of formula (I) in which the different constituents X and Y, or Y and Z, or X, Y and Z are in an equimolar ratio, which is to say that each of the constituents is in a weight proportion of 1, such that the sum of the proportions of each of the constituents is equal to 2 in a salt between two constituents X and Y, or Y and Z, or is equal to 3 in a salt between the three constituents X, Y and Z.

13. Compounds of the following general formula (IV):
(X)_{nₐ}, (Y)_{n_{b}}, Z (IV)
in which:
- nₐ and n_{b} represent 0 or 1, with the proviso that when nₐ = 0 then n_{b} = 1, and when n_{b} = 0 then nₐ = 1,
- X represents a natural amino acid,
- Y represents a keto acid of the following formula (II):
R-CO-COOH (II)
in which R represents an alkyl or alcanoic acid group, substituted or not, of 1 to 10 carbon atoms,
- Z represents a polyamine comprising at least two primary, secondary or tertiary amine functions, spaced by a linear or branched hydrocarbon chain of 3 to 10 carbon atoms.

14. Compound according to claim 13 of the general formula (IV) in which:
- X represents a basic amino acid selected from ornithine, arginine, lysine or histidine,
- Y represents a keto acid of the following formula (II):
R-CO-COOH (II)
in which when R represents:
• -CH₃, said keto acid is pyruvic acid,
• -CH₂-CH₃, said keto acid is α-ketobutyric acid,
• -CH(CH₃)₂, said keto acid is α-ketoisovaleric acid,
• -CH(CH₃)-CH₂-CH₃, said keto acid is β-methylvaleric α-keto acid,
• -CH₂-CH(CH₃)₂, said keto acid is α-keto isocaproic acid,
• -(CH₂)₂-COOH, said keto acid is α-keto glutaric acid,
• -(CH₂)₃-COOH, said keto acid is α-keto adipic acid,
- Z represents a polyamine of the following formula (III):
R₁-HN-(CH₂)ₙ-NH-R₂ (III)
in which:
. n represents a whole number comprised between 4 and 5, and
* when n = 4,
.. R₁ and R₂ represent H,
.. or, R₁ represents H and R₂ represents (CH₂)₃NH₂,
.. or, R₁ and R₂ represent (CH₂)₃NH₂,
* when n = 5,
.. R₁ and R₂ represent H,
.. or, R₁ represents H and R₂ represents a group of the formula

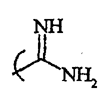

15. Compound according to claim 13 or 14, of the general formula (IV) in which Z represents a polyamine selected from cadaverine, putrescine, spermidine, spermine or agmatine.

16. Compound according to one of claims 13 to 15, of the general formula (IV) in which nₐ = 0 and n_{b} = 1, Y represents α-keto glutaric acid, and Z represents spermidine, namely spermidine α-keto glutarate.

17. Pharmaceutical composition **characterized in that** it comprises a compound according to one of claims 13 to 16, in association with a pharmaceutically acceptable vehicle.
